# EUROPÄISCHE PATENTSCHRIFT

(11) **EP 0 363 507 B1**
(45) Veröffentlichungstag und Bekanntmachung des Hinweises auf die Patenterteilung: **11.08.1993**
(21) Anmeldenummer: 88116873.6
(22) Anmeldetag: 11.10.1988
(51) Int. Cl.: A61B 6/00

(54) **Fahrbares Röntgendiagnostikgerät**
Mobile diagnostic X-ray apparatus
Appareil mobile de radiodiagnostic

(43) Veröffentlichungstag der Anmeldung: 18.04.1990
(73) Patentinhaber: SIEMENS AKTIENGESELLSCHAFT, 80333 München (DE)
(72) Erfinder: Kaul, Karlheinz, Dipl.-Ing.(FH), D-8525 Weiher (DE); Bock, Hans-Christian, Dipl.-Ing. (FH), D-8525 Uttenreuth (DE)

(56) Entgegenhaltungen:
- EP-A- 0 244 561
- DE-A- 3 138 916
- DE-U- 8 521 246
- GB-A- 2 192 591

## Beschreibung

Die Erfindung betrifft ein fahrbares Röntgendiagnostikgerät mit einem Steuerkasten, über dem ein Bedienfeld zum Einstellen und Anzeigen der Aufnahmeparameter für eine Röntgenaufnahme verstellbar ist.

Bei einem bekannten fahrbaren Röntgendiagnostikgerät gemäß der Siemens-Druckschrift "Siremobil 4" ist ein Bedienfeld fest mit einem Steuerkasten verbunden. Dabei ist das Bedienfeld so ausgerichtet, daß es an der hinteren Stirnseite des Röntgendiagnostikgerätes bedienbar ist.

Ein leichtes und komfortables Einstellen und Ablesen der Aufnahmeparameter ist nur von der hinteren Stirnseite des Röntgendiagnostikgerätes möglich.

Ein fahrbares Röntgendiagnostikgerät ist aus dem DE-U-8 521 246 bekannt. Das Fahrwerk dieses Röntgendiagnostikgerätes trägt eine vertikale Säule, an derem oberen Ende ein Auslegerarm um eine vertikale Achse verschwenkbar gelagert ist. Der Auslegerarm trägt einen Bedienungskasten für das Röntgendiagnostikgerät über einem Steuer- und Schaltschrank des Röntgendiagnostikgerätes. Der Bedienungskasten kann somit durch Verschwenken des Auslegerarmes in eine für die Bedienung günstige Position, beisielsweise zur Rückseite oder zu einer Seite des Röntgendiagnostikgerätes, verschwenkt werden. Da der Bedienungskasten durch den Auslegerarm in Augenhöhe gehalten ist, besteht Verletzungsgefahr bei der Bedienung und Handhabung des Röntgendiagnostikgerätes.

Aus der EP-A-0 244 561 ist ein Röntgenuntersuchungstisch bekannt, welcher einen Sockel aufweist, an dem ein Bedienkästchen mit Bedienelementen angeordnet ist. Das Bedienkästchen ist in Tischlängsrichtung am Sockel verschiebbar, so daß es in eine zur Bedienung günstige Position verstellt werden kann.

Aufgabe der Erfindung ist die weitere vorteilhafte Ausgestaltung eines fahrbaren Röntgendiagnostikgerätes der eingangs genannten Art im Hinblick auf komfortable Bedienbarkeit bei geringer Verletzungsgefahr und kostengünstiger Herstellbarkeit.

Die Aufgabe wird erfindungsgemäß dadurch gelöst, daß das Bedienfeld auf der Oberseite des Steuerkastens verstellbar ist und über einen Dauermagnet auf dieser haftet.

Die Aufgabe wird erfindungsgemäß ebenfalls dadurch gelöst, daß das Bedienfeld auf der Oberseite des Steuerkastens verstellbar ist und daß parallel zu beiden Seiten und der hinteren Stirnseite des Steuerkastens in dessen Oberseite Mulden vorgesehen sind, in die das Bedienfeld abgestellt werden kann.

Der Bedienkomfort ist somit wesentlich erhöht, da das Bedienfeld ein sicheres Bedienen und Ablesen der Anzeigen an jeder Seite des Röntgendiagnostikgerätes erlaubt, ohne daß eine Verletzungsgefahr besteht. Die Herstellungskosten sind reduziert, da kein Auslegerarm vorzusehen ist.

Vorteilhafterweise kann das Bedienfeld parallel zu beiden Seiten oder der hinteren Stirnseite des Steuerkastens auf diesem angeordnet werden.

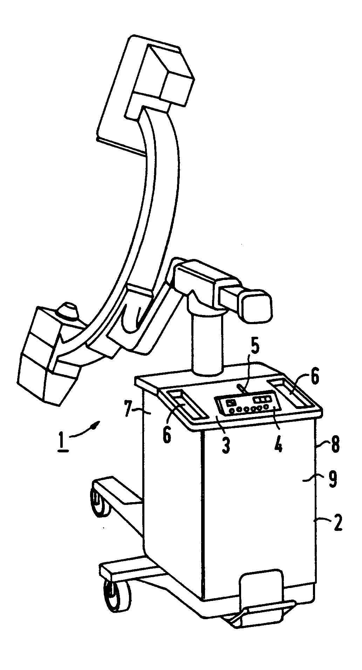

Vorteile und Einzelheiten der Erfindung ergeben sich aus der nachfolgenden Beschreibung eines Ausführungsbeispiels anhand der Zeichnung in Verbindung mit den Unteransprüchen.

Die Figur zeigt ein fahrbares Röntgendiagnostikgerät 1 mit einem Steuerkasten 2, auf dessen Oberseite 3 ein Bedienfeld 4 verstellbar angeordnet ist. Das Bedienfeld 4 ist über ein Kabel 5 mit dem Steuerkasten 2 verbunden. Das Kabel 5 dient neben der freien Verstellbarkeit des Bedienfeldes 4 zur Sicherung gegen Herunterfallen. Im unteren Bereich des Bedienfeldes 4 können beispielsweise Dauermagnete angebracht sein, mit denen das Bedienfeld 4 auf der Oberseite 3 des Steuerkastens 2 haftet. Vorteilhaft können aber auch Mulden 6 parallel zu beiden Seiten 7,8 und der hinteren Stirnseite 9 des Steuerkastens 2 in dessen Oberseite 3 vorgesehen sein, in die das Bedienfeld 4 abgestellt werden kann.

## Patentansprüche

1. Fahrbares Röntgendiagnostikgerät (1) mit einem Steuerkasten (2), über dem ein Bedienfeld (4) zum Einstellen und Anzeigen der Aufnahmeparameter für eine Röntgenaufnahme verstellbar ist,
**dadurch gekennzeichnet**,
daß das Bedienfeld (4) auf der Oberseite (3) des Steuerkastens (2) verstellbar ist und über einen Dauermagnet auf dieser haftet.

2. Fahrbares Röntgendiagnostikgerät (1) mit einem Steuerkasten (2), über dem ein Bedienfeld (4) zum Einstellen und Anzeigen der Aufnahmeparameter für eine Röntgenaufnahme verstellbar ist,
**dadurch gekennzeichnet,**
daß das Bedienfeld (4) auf der Oberseite (3) des Steuerkastens (2) verstellbar ist und
daß parallel zu beiden Seiten (7, 8) und der hinteren Stirnseite (9) des Steuerkastens (2) in dessen Oberseite (3) Mulden (6) vorgesehen sind, in die das Bedienfeld (4) abgestellt werden kann.

3. Fahrbares Röntgendiagnostikgerät (1) nach Anspruch 1 oder 2,
**dadurch gekennzeichnet**,
daß das Bedienfeld (4) über ein Kabel (5) mit dem Steuerkasten (2) in Verbindung steht und daß bei entsprechender Verstellung des Bedienfeldes (4) auf der Oberseite (3) ein Betätigen der Bedientastatur und Ablesen der Anzeigen des Bedienfeldes (4) an den Seiten (7, 8, 9) des Röntgendiagnostikgerätes (1) möglich ist.

4. Fahrbares Röntgendiagnostikgerät (1) nach Anspruch 3,
**dadurch gekennzeichnet,** daß das Kabel (5) das Bedienfeld (4) gegen Herunterfallen sichert.

## Claims

1. Portable X-ray diagnostics apparatus (1) having a control box (2) above which the position of a control panel (4) for setting and indicating the recording parameters for an X-ray recording can be changed, characterized in that the position of the control panel (4) on the upper side (3) of the control box (2) can be changed and the control panel (4) adheres to this upper side by way of a permanent magnet.

2. Portable X-ray diagnostics apparatus (1) having a control box (2) above which the position of a control panel (4) for setting and indicating the recording parameters for an X-ray recording can be changed, characterized in that the position of the control panel (4) on the upper side (3) of the control box (2) can be changed and in that depressions (6) are provided parallel to the two sides (7, 8) and the rear face (9) of the control box (2), in the upper side (3) thereof, in which depressions the control panel (4) can be placed.

3. Portable X-ray diagnostics apparatus (1) according to claim 1 or 2, characterized in that the control panel (4) is connected to the control box (2) by way of a cable (5) and in that, when the position of the control panel (4) on the upper side (3) is changed in an appropriate manner, the control keyboard can be actuated and the displays on the control panel (4) can be read off at the sides (7, 8, 9) of the X-ray diagnostics apparatus (1).

4. Portable X-ray diagnostics apparatus (1) according to claim 3, characterized in that the cable (5) secures the control panel (4) against falling off.

## Revendications

1. Appareil de radiodiagnostic mobile (1) comportant un boîtier de commande (2), au moyen duquel un panneau de commande (4) peut être déplacé pour le réglage et l'affichage des paramètres d'enregistrement pour une radiographie,
caractérisé par le fait que
le panneau de commande (4) est déplaçable sur la face supérieure (3) du boîtier de commande (2) et est fixé par adhérence à ce dernier par l'intermédiaire d'un aimant permanent.

2. Appareil de radiodiagnostic mobile (1) comportant un boîtier de commande (2), au moyen duquel un panneau de commande (4) peut être déplacé pour le réglage et l'affichage des paramètres d'enregistrement pour une radiographie, caractérisé par le fait que
le panneau de commande (4) est déplaçable sur la face supérieure (3) du boîtier de commande (2), et
que des cavités en forme d'auges (6), dans lesquelles le panneau de commande (4) peut être déposé, sont prévues dans la face supérieure (3) du boîtier de commande (2), parallèlement aux deux côtés (7,8) et aux deux faces frontales arrière (9) de ce botier de commande.

3. Appareil de radiodiagnostic mobile (1) suivant la revendication 1 ou 2, caractérisé par le fait que le panneau de commande (4) est raccordé par un câble (5) au boîtier de commande (2) et que, moyennant un déplacement correspondant du panneau de commande (4) sur la face supérieure (3), un actionnement du clavier de commande et une lecture des affichages du panneau de commande (4) sont possibles sur les côtés (7,8,9) de l'appareil de radiodiagnostic (1).

4. Appareil de radiodiagnostic mobile (1) suivant la revendication 3, caractérisé par le fait que le câble (5) empêche toute chute du panneau de commande (4).
